# EUROPEAN PATENT APPLICATION

(11) **EP 3 373 005 A1**
(43) Date of publication of application: **12.09.2018**
(21) Application number: 16862179.5
(22) Date of filing: 04.11.2016
(51) Int. Cl.: G01N 33/53, G01N 33/531, C07K 16/18

(54) **REAGENT AND METHOD FOR CARDIAC TROPONIN-I ASSAY**

(30) Priority: 05.11.2015 JP 2015217909
(71) Applicant: Fujirebio Inc., Shinjuku-ku Tokyo 163-0410 (JP)
(72) Inventor: TOKUNAGA, Takaaki, Tokyo 163-0410 (JP); KONKO, Kazuyasu, Tokyo 163-0410 (JP)
(74) Representative: Hoffmann Eitle
(86) International application number: PCT/JP2016/082733
(87) International publication number: WO 2017/078119

(57) **Abstract**

The present invention provides a reagent for measuring cardiac troponin I comprising an antibody against cardiac troponin I and a polyanionic macromolecule; and a method for measuring cardiac troponin I comprising measuring an amount of cardiac troponin I by using an antibody against cardiac troponin I in the presence of a polyanionic macromolecule.

## Description

### TECHNICAL FIELD

The present invention relates to a reagent and method for measuring cardiac troponin I.

### BACKGROUND ART

Cardiac troponin I is one of three subunits constituting a cardiac troponin complex that involves in regulation of myocardial contraction. The cardiac troponin I is specifically expressed in hearts, and is released into bloods when cardiomyocytes are injured. Thus, it has been used as a blood marker to diagnose myocardial infarction.

The following techniques have been reported as those related to measurements of cardiac troponin I.

Patent Literature 1 discloses that cardiac troponin I can be stabilized by using a matrix containing a given anionic surfactant (an alkyl group having one sulfonate group).

Patent Literature 2 discloses that divalent cations can be used in an immunoassay for cardiac troponin I.

In addition, the following techniques have been reported as those related to immunoassays for proteins, although they are not related to measurements of cardiac troponin I.

Patent Literature 3 discloses that polyanions are added to serums in order to suppress nonspecific reactions associated with antigen-antibody reactions.

Patent Literature 4 discloses that a dextran compound in which a part of hydroxyl groups are substituted by sulfate ester groups can be used in order to suppress nonspecific reactions that occur in indirect immunoagglutination assays.

### Prior Art Reference

### Patent Literature

Patent Literature 1: WO2006/116005
Patent Literature 2: WO2012/115221
Patent Literature 3: Japanese Patent Application Laid-open No. S57-182169
Patent Literature 4: Japanese Patent No. 3327070

### SUMMARY OF INVENTION

### PROBLEM TO BE SOLVED BY THE INVENTION

Although cardiac troponin I has been used to diagnose myocardial infarction, measurement values of cardiac troponin I may vary depending on the types of blood samples. For example, it is known that when serums and plasmas are used as blood samples, measurement values of cardiac troponin I in serums are not necessarily the same to those in plasmas. In addition, blood collection tubes containing a variety of anticoagulants (for example, heparin, EDTA, and citrate) have been used in medical scenes in order to prepare plasmas; however, measurement values of cardiac troponin I in plasmas may vary depending on the types of anticoagulants used for preparing the plasmas. Accordingly, when an amount of cardiac troponin I is measured to diagnose myocardial infarction, a constant cutoff value of cardiac troponin I is hard to be obtained because it is influenced by the types of blood samples.

Also, since an earlier treatment is a key to improve prognosis of acute myocardial infarction, this disease must be diagnosed promptly. Accordingly, plasmas are generally used to diagnose acute myocardial infarction, because unlike serums, plasmas do not require lengthy processes including agglutination reactions and removals of clots. However, as described above, measurement values of cardiac troponin I may vary depending on the types of anticoagulants used for preparing the plasmas. Accordingly, the types of anticoagulants and the types of blood collection tubes to be used for preparing plasmas are usually specified in measurements of cardiac troponin I to diagnose myocardial infarction. However, if an amount of cardiac troponin I can be measured as a constant value regardless of the types of blood collection tubes, it will be versatile because the types of blood collection tubes will no longer need to be specified, and inadvertent problems, such as misuses of unspecified blood collection tubes can be avoided. Accordingly, a method for measuring an amount of cardiac troponin I as a constant value regardless of the types of blood collection tubes used for preparing plasmas needs to be developed.

### MEANS FOR SOLVING PROBLEM

As a result of intensive study to solve the problems described above, the present inventors found that measuring an amount of cardiac troponin I in the presence of a polyanionic macromolecule can reduce a difference of measurement values of the cardiac troponin I between samples, that is, the problems of above can be solved, and whereby the present invention was completed.

That is, the present invention provides [1] to [11] below.
[1] A reagent for measuring cardiac troponin I, comprising an antibody against cardiac troponin I and a polyanionic macromolecule.
[2] The reagent according to [1], wherein the polyanionic macromolecule contains a group selected from the group consisting of a sulfate group, a sulfonate group, and a carboxylate group.
[3] The reagent according to [1] or [2], wherein the reagent comprises a solution containing the antibody and the macromolecule.
[4] The reagent according to [3], wherein the macromolecule in the solution has a concentration of 0.06 mg/mL or higher but 85 mg/mL or lower.
[5] The reagent according to any of [1] to [4], wherein the antibody is an immobilized antibody.
[6] The reagent according to any of [1] to [5], further comprising an additional antibody against cardiac troponin I.
[7] The reagent according to [6], wherein the additional antibody is a labeled antibody.
[8] A method for measuring cardiac troponin I, comprising measuring an amount of cardiac troponin I in a blood sample by using an antibody against cardiac troponin I in the presence of a polyanionic macromolecule.
[9] The method according to [8], wherein the blood sample is plasma.
[10] The method according to [8] or [9], comprising (1) to (3) below:
   (1) preparing a mixed solution of the antibody against cardiac troponin I, the polyanionic macromolecule, and the blood sample;
   (2) incubating the mixed solution; and
   (3) measuring an amount of cardiac troponin I in the mixed solution.
[11] The method according to [10], wherein the macromolecule in the mixed solution has a concentration of 0.05 mg/mL or higher but 5.0 mg/mL or lower.

### EFFECT OF THE INVENTION

According to the present invention, a difference of measurement values of cardiac troponin I between samples can be reduced. Accordingly, the present invention provides the following advantages: when an amount of cardiac troponin I is measured to diagnose myocardial infarction, a constant cutoff value of cardiac troponin I can easily be employed regardless of the types of blood samples; and an amount of cardiac troponin I can be measured as a constant value regardless of the types of blood collection tubes used for preparing plasmas.

### BRIEF DESCRIPTION OF DRAWINGS

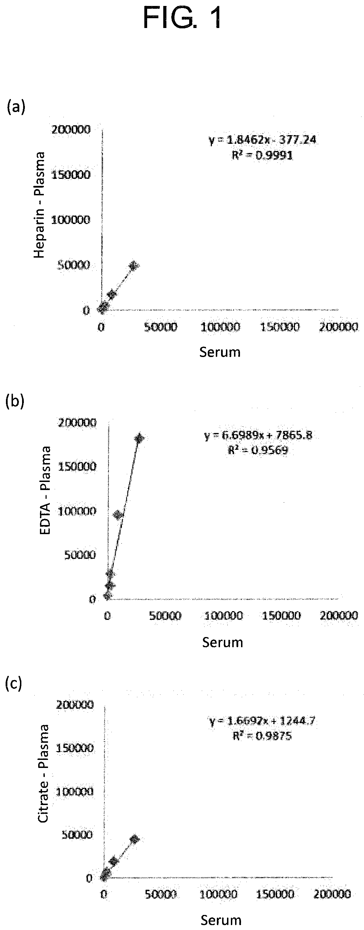

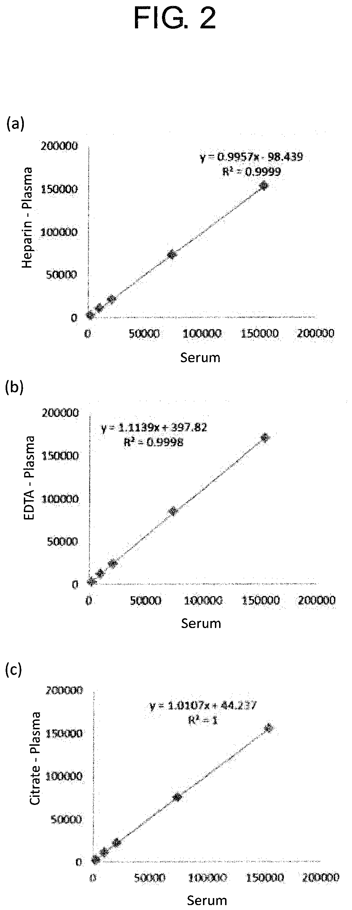

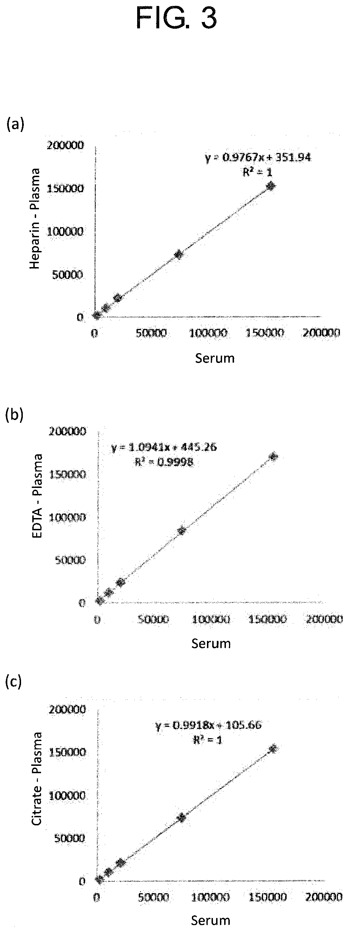

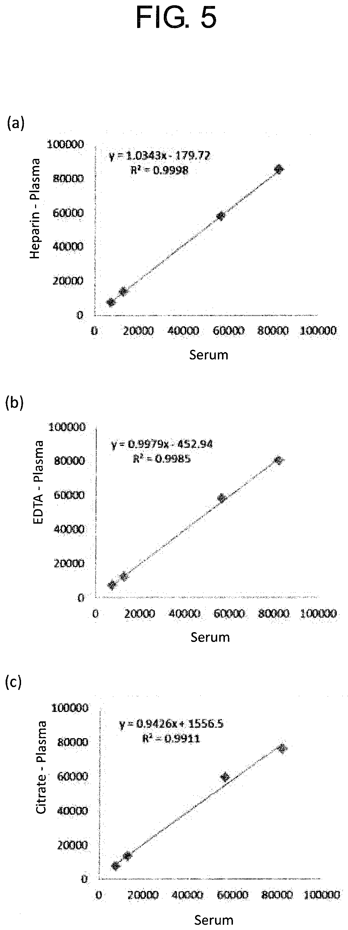

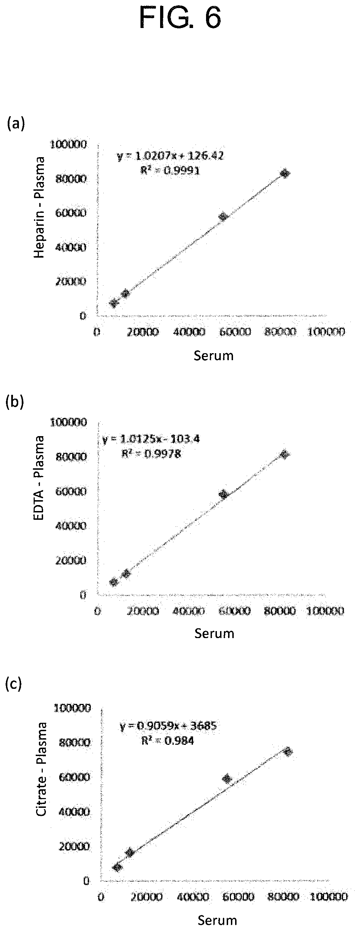

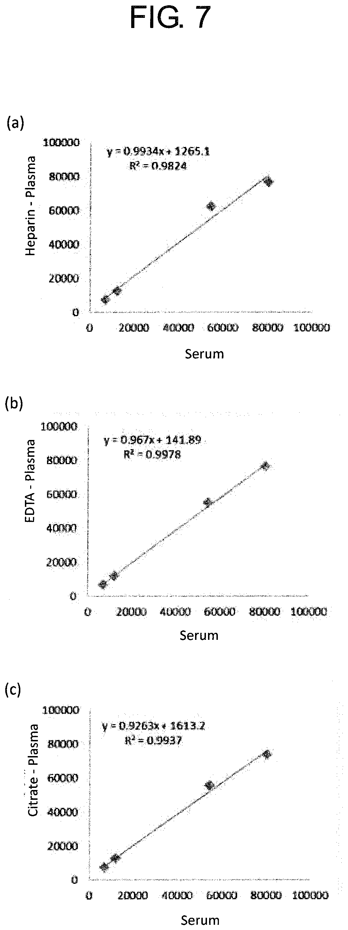

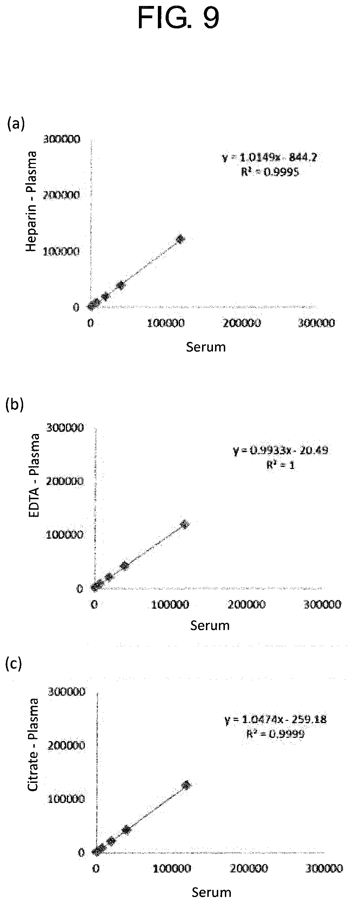

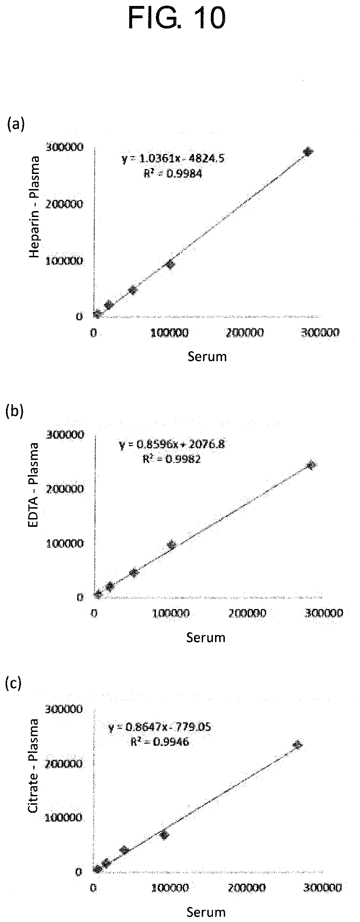

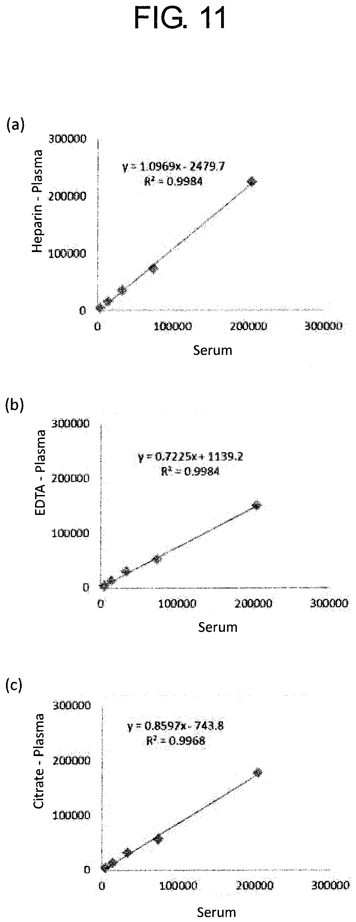

FIG. 1 is a graph illustrating correlation of luminescence intensities among serums and plasmas in measurements of cardiac troponin I using Reagent A described in Example 1 in the absence of dextran sulfate sodium. (a) Correlation of luminescence intensities among serums and heparin plasmas; (b) correlation of luminescence intensities among serums and EDTA plasmas; and (c) correlation of luminescence intensities among serums and citrate plasmas (also applied to FIGs. 2 to 11). As the slope is closer to 1, the difference of measurement values among serums and plasmas will be smaller. This indicates that a difference of measurement values among serums and plasmas are more reduced (also applied to FIGs. 2 to 11).
FIG. 2 is a graph illustrating correlation of luminescence intensities among serums and plasmas in measurements of cardiac troponin I using an antibody-conjugated particle solution (Reagent A) containing 0.77 mg/mL dextran sulfate sodium.
FIG. 3 is a graph illustrating correlation of luminescence intensities among serums and plasmas in measurements of cardiac troponin I using the antibody-conjugated particle solution (Reagent A) containing 1.55 mg/mL dextran sulfate sodium.
FIG. 4 is a graph illustrating correlation of luminescence intensities among serums and plasmas in measurements using Reagent B described in Example 1 in the absence of dextran sulfate sodium.
FIG. 5 is a graph illustrating correlation of luminescence intensities among serums and plasmas in measurements of cardiac troponin I using an antibody-conjugated particle solution (Reagent B) containing 0.5 mg/mL dextran sulfate sodium.
FIG. 6 is a graph illustrating correlation of luminescence intensities among serums and plasmas in measurements of cardiac troponin I using the antibody-conjugated particle solution (Reagent B) containing 1.0 mg/mL dextran sulfate sodium.
FIG. 7 is a graph illustrating correlation of luminescence intensities among serums and plasmas in measurements of cardiac troponin I using the antibody-conjugated particle solution (Reagent B) containing 2.0 mg/mL dextran sulfate sodium.
FIG. 8 is a graph illustrating correlation of luminescence intensities among serums and plasmas in measurements of cardiac troponin I using the antibody-conjugated particle solution (Reagent B) containing 1.5 mg/mL dextran sulfate sodium.
FIG. 9 is a graph illustrating correlation of luminescence intensities among serums and plasmas in measurements of cardiac troponin I using the antibody-conjugated particle solution (Reagent B) containing 1.5 mg/mL sodium polystyrene sulfonate.
FIG. 10 is a graph illustrating correlation of luminescence intensities among serums and plasmas in measurements of cardiac troponin I using the antibody-conjugated particle solution (Reagent B) containing 1.5 mg/mL sodium polyacrylate.
FIG. 11 is a graph illustrating correlation of luminescence intensities among serums and plasmas in measurements of cardiac troponin I using the antibody-conjugated particle solution (Reagent B) containing 1.5 mg/mL of sodium N-lauroyl sarcosinate.

### EMBODIMENTS FOR CARRYING OUT THE INVENTION

### <1. Reagent of Present Invention>

The present invention provides a reagent for measuring cardiac troponin I comprising an antibody against cardiac troponin I and a polyanionic macromolecule.

The cardiac troponin I (cTnI) measured with reagents of the present invention is one of three subunits (troponin I, C and T) constituting a cardiac troponin complex that relates to control of myocardial contraction. Although the cardiac troponin I measured with the present invention may be cardiac troponin I derived from any animals, it is preferably cardiac troponin I derived from mammals (for example, primates such as humans, monkeys, and chimpanzees; rodents such as mice, rats, and rabbits; pets such as dogs and cats; domestic animals such as pigs and cattle; and working animals such as horses and sheep), more preferably cardiac troponin I derived from primates, and particularly preferably cardiac troponin I derived from humans. For an example of an amino acid sequence of cardiac troponin I derived from humans, see GenBank: CAA62301.1. Of course the cardiac troponin I derived from humans is not limited to the one consisting of the amino acid sequence referring to the above number, and it may be a variant thereof (for example, a naturally occurring variant). In addition, the cardiac troponin I measured with the present invention may be any of free forms, complex forms with troponin C and/or troponin T, and complex forms with other molecules such as autoantibodies.

The antibody against cardiac troponin I contained in the reagent of the present invention recognizes at least a portion of an amino acid sequence of cardiac troponin I as epitopes. As epitopes recognized by the antibody against cardiac troponin I, a variety of epitopes including specific epitopes have been known (see, for example, Filatov vl et al., Biochem. Mol. Biol. Int. 1998, 45 (6): 1179-1187; WO2012/115221). Accordingly, the antibody against cardiac troponin I is not particularly limited, and it may be an antibody recognizing such a variety of epitopes; however, the epitope is preferably the one widely used in clinical tests of cardiac troponin I using antibodies against cardiac troponin I. In amino acid sequences of cardiac troponin I derived from humans, examples of the epitopes include epitopes found in peptide portions consisting of the 20th to 60th amino acid residues (for example, peptides consisting of amino acid residues of the 24th to 40th, or the 41st to 49th), epitopes found in peptide portions consisting of the 61st to 120th amino acid residues (for example, peptides consisting of amino acid residues of the 86th to 90th), epitopes found in peptide portions consisting of the 130th to 150th amino acid residues, and epitopes found in peptide portions consisting of the 160th to 209th amino acid residues. The antibody against cardiac troponin I preferably recognizes cardiac troponin I specific epitopes (in particular, human cardiac troponin I specific epitopes).

The antibody against cardiac troponin I may be a polyclonal antibody or a monoclonal antibody. The antibody against cardiac troponin I may be any of the immunoglobulin isotypes (for example, IgG, IgM, IgA, IgD, IgE, and IgY). The antibody against cardiac troponin I may be a full length antibody. The full length antibody refers to an antibody including heavy chains and light chains in which each heavy chain and light chain has variable regions and constant regions (for example, an antibody having two Fab portions and an Fc portion). The antibody against cardiac troponin I may also be an antibody fragment derived from such a full length antibody. The antibody fragment is a portion of such a full length antibody, and an example thereof is a constant region-deleted antibody (for example, F(ab')₂, Fab', and Fab, Fv). The antibody against cardiac troponin I may also be a modified antibody such as a single chain antibody.

The antibody against cardiac troponin I can be prepared by using a conventionally known method. For example, the antibody against cardiac troponin I can be prepared by using an epitope of above as an antigen. Also, a variety of antibodies against cardiac troponin I, which recognize the epitopes described above, are commercially available to be used.

The antibody against cardiac troponin I may be immobilized on a solid phase. In the present specification, an antibody immobilized on a solid phase may simply be referred to as an immobilized antibody. Examples of the solid phase include solid phases that can accommodate or load liquid phases (for example, supports such as plates, membranes, and test tubes; and chambers such as well plates, microfluidic path, glass capillaries, nanopillars, and monolith columns) and solid phases that can be suspended or dispersed in liquid phases (for example, solid phase carriers such as particles). Examples of solid phase materials include glass, plastics, metals, and carbons. In addition, although nonmagnetic materials or magnetic materials can be used as solid phase materials, magnetic materials are preferred because of convenience handling and/or the like. Solid phases are preferably solid phase carriers, more preferably magnetic solid phase carriers, and still more preferably magnetic particles. In order to make antibodies to be immobilized, conventionally known methods can be utilized. Examples of such methods include physical adsorption methods, covalent bond methods, methods using affinity substances (for example, biotin and streptavidin), and ionic bond methods. In a specific embodiment, the antibody against cardiac troponin I is an antibody immobilized on a solid phase, preferably an antibody immobilized on a magnetic solid phase, and more preferably an antibody immobilized on magnetic particles.

The antibody against cardiac troponin I may be labeled with a labeling substance. In the present specification, an antibody labeled with a labeling substance may simply be referred to as a labeled antibody. Examples of the labeling substance include enzymes (for example, peroxidase, alkaline phosphatase, luciferase, and β galactosidase), affinity substances (for example, streptavidin and biotin), fluorescent substances or proteins (for example, fluorescein, fluorescein isothiocyanate, rhodamine, green fluorescent proteins, and red fluorescent proteins), luminescent or light-absorbing substances (for example, luciferin, aequorin, and acridinium), and radioactive substances (for example, ³H, ¹⁴C, ³²P, ³⁵S, and ¹²⁵I). In addition, when a second antibody (for example, an additional antibody mentioned later) is used in the method of the present invention, the second antibody may be labeled with such a labeling substance.

Polyanionic macromolecules are also included in the reagents of the present invention.

In the present invention, the term "polyanionic macromolecule" refers to a macromolecule containing a plurality of anionic portions. The term "anionic portion" refers to a negatively charged group or atom. Examples of the anionic portion include a sulfate group [-O-S(=O)₂-O⁻], a sulfonate group [-S(=O)₂-O⁻], a carboxylate group [-C(=O)-O⁻], a phosphate group [-O-P(=O)(-O⁻)₂], a hydrogen phosphate group [-O-P(=O)(-OH)(-O⁻)], a negatively charged sulfur atom [-S⁻], and a negatively charged oxygen atom group [-O⁻]. Preferably, the anionic portion is a group selected from the group consisting of a sulfate group, a sulfonate group, and a carboxylate group. The polyanionic macromolecule may contain one type or two types or more of anionic portions. Here, the number of anionic portions contained in the polyanionic macromolecule is not particularly limited as long as a difference of measurement values of the cardiac troponin I levels can be reduced. Although the number varies depending on the types of anionic portions, it is usually 5 or more, preferably 15 or more, and more preferably 30 or more. The number of anionic portions contained in the polyanionic macromolecule may be 500 or less, 400 or less, or 300 or less. A molecular weight of the polyanionic macromolecule is not particularly limited as long as a difference of measurement values of the cardiac troponin I levels can be reduced. Although the molecular weight varies depending on types and numbers of anionic portions as well as the types of polyanionic macromolecules, it is usually 500 or higher, preferably 1,000 or higher, and more preferably 3,000 or higher. Also, a molecular weight of the polyanionic macromolecule may be 100,000 or lower, 70,000 or lower, or 50,000 or lower. Note that when the polyanionic macromolecule is a polyanionic polymer as described later, the molecular weight refers to a weight-average molecular weight unless otherwise noted.

The polyanionic macromolecule may be a form of salt. Examples of the salt include inorganic salts and organic salts. Examples of the inorganic salt include ammonium salts and metal salts. Examples of the metal salt include monovalent metal salts, such as sodium salts and potassium salts; and divalent metal salts, such as calcium salts and magnesium salts. Examples of the organic salt include ammonium salts substituted by alkyl groups and nitrogen-containing heterocyclic compound salts (for example, pyridinium salts).

In a preferable embodiment, the polyanionic macromolecule may be a polymer having a repeating unit containing one or two or more of anionic portions as described above. In the present invention, the polymer having a repeating unit containing one or two or more of anionic portions may be referred to as "polyanionic polymer." The polyanionic polymer may have structural units other than the repeating unit containing one or two or more of anionic portions. Accordingly, the polyanionic polymer may be a homopolymer or a copolymer (for example, a block copolymer). Also, the polyanionic polymer may be a linear polymer, a polymer having a branched structure, or a dendrimer. In addition, although the polyanionic polymer may be a polymer obtained by polymerizing monomers containing anionic portions (if necessary, block units with other structural units), or a polymer obtained by introducing a plurality of anionic portions into a polymer not containing an anionic portion (for example, a dextran compound in which hydroxyl groups have been substituted by sulfate esters as described in Japanese Patent No. 3327070), it is preferably a polymer obtained by polymerizing monomers containing anionic portions. The polyanionic polymer may be a form of salt.

In the present invention, although the number of repeating units in the polyanionic polymer is not particularly limited as long as a difference of measurement values of the cardiac troponin I levels can be reduced, it may be, for example, 5 or more, 10 or more, 15 or more, 20 or more, 30 or more, or 40 or more. In the polyanionic polymer, the number of repeating units containing anionic portions may be, for example, 500 or less, 400 or less, or 300 or less.

Specific examples of the polyanionic polymer include polysulfate compounds (for example, dextran sulfate and chondroitin sulphate), polysulfonate compounds (for example, polystyrene sulfonate and polyvinyl sulfonate), polycarboxylate compounds (for example, polyacrylate, polymethacrylate, polymaleate, and polyfumarate), polyanionic polysaccharides (for example, dextran sulfate, carboxymethyl dextran, carrageenan, and xanthan gum), and polyanionic proteins (for example, polyaspartate and polyglutamate). Preferably, the polyanionic polymer may be dextran sulfate, polystyrene sulfonate, or polyacrylate.

The polyanionic macromolecule can be prepared by a conventionally known method. For example, it can be prepared by a method in which monomers containing anionic portions are polymerized or a method in which anionic portions are introduced in macromolecules not containing anionic portions. A commercially available polyanionic macromolecule may be used.

Also, the reagent of the present invention may further contain an additional antibody other than the antibody against cardiac troponin I and the polyanionic polymer described above. Examples of the additional antibody include an additional antibody against cardiac troponin I, which recognizes an epitope different from the epitope recognized by the antibody against cardiac troponin I described above; an antibody that recognizes a constant region of an antibody against cardiac troponin I; and an antibody that recognizes a complex of an antibody against cardiac troponin I and cardiac troponin I. Such an additional antibody can be used, for example, as a second antibody.

In a specific embodiment, the reagent of the present invention contains an additional antibody against cardiac troponin I, which recognizes an epitope different from the epitope recognized by the antibody against cardiac troponin I, as an additional antibody. Details of the epitope recognized by such an additional antibody are same to those of the epitope recognized by the antibody against cardiac troponin I (however, for combination uses, the types of the epitopes are different). A combination of an epitope recognized by an antibody against cardiac troponin I and an epitope recognized by an additional antibody against cardiac troponin I is not particularly limited. For example, when an antibody that recognizes a specific epitope found in peptide portions consisting of the 20th to 60th amino acid residues (for example, peptides consisting of amino acid residues of the 24th to 40th, or the 41st to 49th) is used as an antibody against cardiac troponin I, an antibody that recognizes an epitope other than the specific epitope, for example, another epitope found in peptide portions consisting of the 20th to 60th amino acid residues (for example, peptides consisting of amino acid residues of the 24th to 40th, or the 41st to 49th), another epitope found in peptide portions consisting of the 61st to 120th amino acid residues (for example, peptides consisting of amino acid residues of the 86th to 90th), another epitope found in peptide portions consisting of the 130th to 150th amino acid residues, or another epitope found in peptide portions consisting of the 160th to 209th amino acid residues can be used as an additional antibody against cardiac troponin I. Such an additional antibody is preferably used in a case where, for example, a sandwich method is used.

Moreover, the reagent of the present invention may contain a component other than the substances described above. Examples of such a component include buffers or diluents (for example, MES buffer, phosphate buffer, Tris buffer, and carbonate buffer), the labeling substance described above, and a substrate reacted with the labeling substance (for example, when the labeling substance is an enzyme, the substrate of the enzyme). Although pH of the buffer is same to the pH usually used for a buffer containing an antibody, when a buffer containing an antibody against cardiac troponin I is used, pH of the buffer must be the pH that can maintain the negatively charged status of anionic portions in a polyanionic macromolecule. Such pH varies depending on the types of the polyanionic macromolecule, and it is for example 5 to 9, preferably 5.6 to 7.6.

The reagent of the present invention can be used in an immunoassay in which an antibody against cardiac troponin I is used. Examples of such an immunoassay include a direct competitive method, an indirect competitive method, and a sandwich method. Examples of such an immunoassay also include chemiluminescence immunoassay (CLIA) (for example, chemiluminescence enzyme immunoassay (CLEIA)), turbidimetric immunoassay (TIA), enzyme immunoassay (EIA) (for example, direct competitive ELISA, indirect competitive ELISA, and sandwich ELISA), radioimmunoassay (RIA), latex agglutination reaction method, fluorescent immunoassay (FIA), and immunochromatography.

The reagent of the present invention contains components in a manner which are separated each other, or are in a composition. Specifically, although each of the components may be provided as accommodated in a separated container (for example, a tube and a plate), some components may be provided as a composition (for example, in one solution). Alternatively, the reagent of the present invention may be provided as a form of a device. Specifically, it may be provided such that all of the components are accommodated in a device. Alternatively, it may be provided such that a part of components is accommodated in a device, and remaining is not accommodated in the device (for example, accommodated in a different container). In this case, the components not accommodated in the device may be used by being injected into the device when a target substance is measured.

In a specific embodiment, the reagent of the present invention containing the antibody against cardiac troponin I and the polyanionic macromolecule may be provided as a form of kit including a solution or powder of the antibody against cardiac troponin I and a solution or powder of the polyanionic macromolecule, which are contained in a same container or each of which is contained in different container; however, the reagent may be provided as a form of one solution containing cardiac troponin I and the antibody against cardiac troponin I (premix) in terms of avoidance of preparations at times of uses and/or the like. When the polyanionic macromolecule is provided as a solution in the reagent of the present invention, a concentration of the polyanionic macromolecule in the solution is, for example, 0.06 mg/mL or higher, but 85 mg/mL or lower, although the concentration varies depending on conditions of uses, such as a mixing ratio to a sample to be analyzed and a dilution ratio. A concentration of the polyanionic macromolecule in the solution may be preferably 0.08 mg/mL or higher, more preferably 0.2 mg/mL or higher, still more preferably 0.8 mg/mL or higher. A concentration of the polyanionic macromolecule in the solution may also be preferably 8.5 mg/mL or less, more preferably 4.0 mg/mL or lower, still more preferably 3.0 mg/mL or lower.

In a preferable embodiment, the reagent of the present invention may have configurations depending on the types of immunoassays to be used. For example, when a sandwich method is used, the reagent of the present invention may contain i) an antibody against cardiac troponin I and ii) a polyanionic polymer, as essential components; iii) an additional antibody against cardiac troponin I, iv) a labeling substance, and v) a diluent (buffer), as optional components; and vi) a substrate reacted with the labeling substance, if necessary. The components of i) and ii) may be contained in one solution. The component of iii) may be labeled with a labeling substance of iv). Preferably, the antibody against cardiac troponin I may be immobilized on magnetic particles. A specific example of the configuration of the reagent of the present invention is i') a buffer containing magnetic particles on which an antibody against cardiac troponin I is immobilized and a polyanionic polymer, ii') a buffer containing an additional antibody against cardiac troponin I (labeled with a labeling substance), and iii') a diluent (buffer).

The reagent of the present invention is useful as, for example, a testing agent using blood samples, because the reagent can measure amounts of cardiac troponin I in blood samples as constant values regardless of the types of blood samples. As is clear from the fact that amounts of cardiac troponin I in blood samples can be measured as constant values regardless of the types of blood samples by using the reagent of the present invention, it is thought that an interaction between cardiac troponin I and an antibody can be stabilized by eliminating influence of various contaminants in samples. Accordingly, it is thought that the reagent is excellent for measuring amounts of cardiac troponin I in samples other than blood samples, which contain various contaminants. As the samples such as blood samples, samples subjected to preliminary processing may be used. Examples of such preliminary processing include centrifugation, fractionation, extraction, filtration, precipitation, heating, freezing, refrigeration, and stirring.

In a preferable embodiment, the reagent of the present invention can be used as an agent for diagnosing diseases (for example, acute myocardial infarction and myocarditis). A preferable sample is a blood sample.

For the blood samples, any kind of blood samples can be used, and examples of the blood sample include serums and plasmas. For the plasmas, those treated with anticoagulants (for example, plasmas collected in blood collection tubes containing anticoagulants) can be used. Examples of anticoagulants include, but not limited to, heparin, EDTA, citrate, and salts thereof; and sodium fluoride. For the blood samples, those derived from any animals can be used, preferably blood samples are derived from mammals described above, and more preferably, derived from primates described above. Human blood samples are particularly preferred because of clinical applications to humans.

An EDTA plasma usually used is prepared by adding an appropriate amount (for example, about 1.5 mg/mL or about 2.0 mg/mL for disodium EDTA; or about 1.8 mg/mL, about 1.85 mg/mL, or about 1.9 mg/mL for dipotassium EDTA) of EDTA salt to a whole blood sample, inverting and mixing the sample, and centrifuging the sample to remove blood cell components. A citrate plasma usually used is prepared by adding an appropriate amount (for example, about 3.2 mg/mL, if it is sodium citrate) of citrate salt to a whole blood sample, inverting and mixing the sample, and centrifuging the sample to remove blood cell components. A heparin plasma usually used is prepared by adding an appropriate amount (for example, about 13 IU/mL, if it is heparin sodium) of heparin salt to a whole blood sample, inverting and mixing the sample, and centrifuging the sample to remove blood cell components.

The reagent of the present invention can reduce the difference of measurement values for cardiac troponin I among various blood samples. A rate of the difference of measurement values between the various blood samples (a percentage of an absolute value of the difference between a measurement value "a" and a measurement value "b" |a-b| to the measurement value "a" (|a-b|/a)×100(%), in which "a" is a measurement value of a standard blood sample (for example, a serum), and "b" is a measurement value of a blood sample compared to the standard blood sample (for example, an EDTA plasma, a citrate plasma, and a heparin plasma)) is preferably less than 15%, more preferably less than 10%, still more preferably less than 5%, still more preferably less than 1%, and particularly preferably less than 0.5%.

### <2. Method of Present Invention>

The present invention also provides a method for measuring cardiac troponin I. The method of the present invention include measuring an amount of cardiac troponin I in a blood sample by using an antibody against cardiac troponin I in the presence of a polyanionic macromolecule. Definitions, examples, and preferable examples of cardiac troponin I, the antibody against cardiac troponin I, the polyanionic macromolecule, and the blood sample are described above.

An amount of the polyanionic macromolecule used in the method of the present invention should be an amount that can reduce a difference of measurement values of cardiac troponin I between the types of blood samples.

An amount of cardiac troponin I can be measured by, for example, immunoassays described above. Among them, a sandwich method and/or a chemiluminescence enzyme immunoassay (CLEIA) are/is preferred, but not limited thereto.

Specifically, the method of the present invention may include (1) to (3) below:
(1) preparing a mixed solution of an antibody against cardiac troponin I, a polyanionic macromolecule, and a blood sample;
(2) incubating the mixed solution; and
(3) measuring an amount of cardiac troponin I in the mixed solution.

In Step (1), the mixed solution can be prepared by properly mixing a solution or powder of an antibody against cardiac troponin I, a solution or powder of a polyanionic macromolecule, and a blood sample; and a diluent, if necessary. Examples of the solution or diluent include water (for example, distilled water, sterilized water, sterilized distilled water, and pure water), and buffers described above. Among them, buffers are preferred. It may be preferable that a solution containing an antibody against cardiac troponin I and a polyanionic macromolecule is prepared in advance, and the solution thus prepared is mixed with a blood sample, and if necessary, with a diluent.

A concentration of the polyanionic macromolecule in the mixed solution is not particularly limited, as long as a difference of measurement values of the cardiac troponin I levels depending on the types of blood samples can be reduced. Although the concentration varies depending on the types of samples, it is for example 0.05 mg/mL or higher, but 5.0 mg/mL or lower. The concentration of the polyanionic macromolecule in the mixed solution may preferably be 0.1 mg/mL or higher, more preferably 0.3 mg/mL or higher, and still more preferably 0.5 mg/mL or higher. The concentration of the polyanionic macromolecule in the mixed solution may also preferably be 2.0 mg/mL or lower, more preferably 1.5 mg/mL or lower, and still more preferably 1.0 mg/mL or lower.

In Step (2), the mixed solution can be incubated for sufficient time to form a complex of cardiac troponin I, which may exist in a blood sample, and an antibody against cardiac troponin I, at an appropriate temperature. Such time is similar to that adapted in usual immunoassays, and is, for example, 1 minute to 24 hours. Such a temperature is similar to that adapted in usual immunoassays, and is, for example, 5°C to 40°C.

In Step (3), an amount of cardiac troponin I in the mixed solution can be measured in the immunoassay described above by using an antibody against cardiac troponin I. By measuring an amount of cardiac troponin I in the mixed solution, an amount of cardiac troponin I existing in a blood sample can be evaluated.

The method of the present invention may include a step of preliminary processing. Examples of such a step include centrifugation, fractionation, extraction, filtration, precipitation, heating, freezing, refrigeration, and stirring.

The method of the present invention is useful for, for example, tests using blood samples, because the method can measure amounts of cardiac troponin I in blood samples as constant values regardless of the types of the blood samples. The method of the present invention can preferably be used to diagnose the diseases as described above.

### EXAMPLES

Hereinafter, the present invention will be explained in detail with examples; however, the present invention is not limited to these examples.

### [Example 1] Preparation and Measurement Method of Reagent for Measuring Cardiac Troponin I, and Preparation of Test Sample

As reagents for measuring cardiac troponin I, Reagent A and Reagent B were prepared. A pair of antibodies used in Reagent A was different from that used in Reagent B.

### <Reagent A>

- Antibody-conjugated particle solution (a solution of immobilized antibodies): An antibody-conjugated particle solution (pH 6.8) containing antibody-conjugated magnetic particles in which a mouse monoclonal antibody has been conjugated to 0.025% (w/v) carboxylated magnetic particles (manufactured by FUJIREBIO Inc.), which recognizes the 41st to 49th amino acid sequence of cardiac troponin I (see, for example, GenBank: CAA62301.1, the same applies hereinafter) as an epitope; 50 mM of 2-morpholino ethanesulfonate (MES); 1.0% (w/v) of bovine serum albumin (BSA); and 50 mM of NaCl was prepared.
- Labeled antibody solution: A labeled antibody solution (pH 6.8) containing a labeled antibody obtained by labeling 0.5 µg/mL of an antibody, which recognizes the 86th to 90th amino acid sequence of cardiac troponin I as an epitope, with an alkaline phosphatase (a recombinant having high specific activity and the reduced degree of sugar chains, manufactured by Roche Diagnostics K.K.); 50 mM of MES; 2.5% (w/v) of BSA; 100 mM of NaCl; 0.3 mM of ZnCl₂; and 1.0 mM of MgCl₂ was prepared.

These solutions were packed in a cartridge for the automated immunoassay system (Lumipulse G1200, manufactured by FUJIREBIO Inc.).

### <Reagent B>

- Antibody-conjugated particle solution (a solution of immobilized antibodies): An antibody-conjugated particle solution (pH 6.8) containing antibody-conjugated magnetic particles in which a mouse monoclonal antibody has been conjugated to 0.025% (w/v) carboxylated magnetic particles (manufactured by FUJIREBIO Inc.), which recognizes the 24th to 40th amino acid sequence of cardiac troponin I as an epitope; 50 mM of 2-morpholino ethanesulfonate (MES); 1.0% (w/v) of BSA; and 50 mM of NaCl was prepared.
- Labeled antibody solution: A labeled antibody solution (pH 6.8) containing a labeled antibody obtained by labeling 0.5 µg/mL of an antibody, which recognizes the 41st to 49th amino acid sequence of cardiac troponin I as an epitope, with an alkaline phosphatase (a recombinant having high specific activity and reduced degree of sugar chains, manufactured by Roche Diagnostics K.K.); 50 mM of MES; 2.5% (w/v) of BSA; 100 mM of NaCl; 0.3 mM of ZnCl₂; and 1.0 mM of MgCl₂ was prepared.

These solutions were packed in a cartridge for the automated immunoassay system (Lumipulse G1200, manufactured by FUJIREBIO Inc.).

Amounts of cardiac troponin I of test samples were measured by using the automated immunoassay system (Lumipulse G1200, manufactured by FUJIREBIO Inc.) according to the procedure described below.
(1) Adding 100 µL of a test sample to 150 µL of an antibody-conjugated particle solution to prepare a first reaction solution. Stirring the first reaction solution, and then incubating it at 37°C for 10 minutes in order to form an immune complex of an anti-cardiac troponin I antibody bound on magnetic particles and a cardiac troponin I antigen contained in the test sample.
(2) After the incubation, gathering magnetic particles on a tube wall with a magnet, and substances not attached on the magnetic particles are removed. Then, repeating additions and removals of a wash solution (Lumipulse (registered trademark) Wash Solution, manufactured by FUJIREBIO Inc.) to wash the magnetic particles.
(3) After washing, mixing 250 µL of the labeled antibody solution and magnetic particles to prepare a second reaction solution. Incubating the second reaction solution at 37°C for 10 minutes in order to form an immune complex composed of an anti-cardiac troponin I antibody immobilized on magnetic particles-an cardiac troponin I antigen-an anti-cardiac troponin I antibody labeled with an alkaline phosphatase.
(4) After the incubation, gathering magnetic particles again on a tube wall with a magnet, and substances not attached on the magnetic particles are removed. Then, repeating additions and removals of the wash solution to wash the magnetic particles.
(5) Adding 200 µL of a substrate solution containing AMPPD (3-(2'-spiroadamantane)-4-methoxy-4-(3'-phosphoryloxy)phenyl-1,2-dioxetane disodium salt) (Lumipulse (registered trademark) Substrate Solution, manufactured by FUJIREBIO Inc.) to magnetic particles. Stirring the mixture, and then incubating it at 37°C for 5 minutes. The AMPPD contained in the substrate solution is degraded by the catalysis of an alkaline phosphatase indirectly bound on magnetic particles to emit light having an emission maximum at a wavelength of 477 nm. A luminescence intensity reflects an amount of cardiac troponin I bound on magnetic particles, and thus an amount of cardiac troponin I can be measured by measuring a luminescence intensity at a wavelength of 477 nm.

For preparation of test samples, 4 or 5 samples having higher values of cardiac troponin I (manufactured by ProMedDx, LLC) and 4 or 5 paired serum and plasma sample (a serum, an EDTA plasma, a citrate plasma, and a heparin plasma obtained from an identical donor) were provided. The test samples were prepared by adding the sample having a higher value of cardiac troponin I to the paired serum and plasma sample so that a volume of the sample having a higher value of cardiac troponin I was 1/10 or lower of that of the paired serum and plasma sample.

### [Example 2] Effects of Dextran Sulfate Sodium to Correlation between Serum and Plasma on Measurement of Cardiac Troponin I

Dextran sulfate sodium (Dextran sulfate sodium 5000, manufactured by Wako Pure Chemical Industries, Ltd.) was added to an antibody-conjugated particle solution of Reagent A so that the concentration was 0 mg/mL, 0.77 mg/mL, 1.55 mg/mL, or 2.32 mg/mL (each of the concentrations in the first reaction solution was 0 mg/mL, 0.462 mg/mL, 0.930 mg/mL, or 1.392 mg/mL), and then the test samples were measured. The results are listed in Table 1 and illustrated in FIGs. 1 to 3. Each of the measurement values is a luminescence intensity at a wavelength of 477 nm (count value). A count value to serum (average) was obtained for each of the samples by calculating the percentage of a count value of each of plasmas to a count value of serum, followed by calculating the average value of them. The count values to serum (averages) shown in Examples 3 and 4 were calculated in a similar way.

**Table 1. Measurement of Cardiac Troponin I Using Reagent A**

| | | Dextran Sulfate Sodium Content (mg/mL) | | | |
|---|---|---|---|---|---|
| Sample | Sample Type | 0 | 0.77 | 1.55 | 2.32 |
| A | Serum | 580 | 2718 | 2741 | 2544 |
| | Heparin Plasma | 785 | 2834 | 2664 | 2541 |
| | EDTA Plasma | 4109 | 2894 | 2694 | 2628 |
| | Citrate Plasma | 915 | 2550 | 2418 | 2509 |
| B | Serum | 2802 | 10995 | 10771 | 10648 |
| | Heparin Plasma | 4163 | 11101 | 10751 | 10822 |
| | EDTA Plasma | 15278 | 12643 | 12167 | 12426 |
| | Citrate Plasma | 4165 | 11183 | 10919 | 10749 |
| C | Serum | 3109 | 22125 | 21570 | 21129 |
| | Heparin Plasma | 5181 | 21912 | 22128 | 21275 |
| | EDTA Plasma | 28764 | 24652 | 24032 | 23851 |
| | Citrate Plasma | 7256 | 22681 | 21861 | 21883 |
| D | Serum | 9090 | 74907 | 74709 | 72539 |
| | Heparin Plasma | 17367 | 73631 | 73047 | 71139 |
| | EDTA Plasma | 95259 | 85512 | 83743 | 83632 |
| | Citrate Plasma | 19606 | 75713 | 74132 | 73505 |
| E | Serum | 27055 | 153857 | 155364 | 152460 |
| | Heparin Plasma | 49334 | 153495 | 152136 | 150409 |
| | EDTA Plasma | 181532 | 171032 | 169706 | 167366 |
| | Citrate Plasma | 45448 | 155536 | 154172 | 151726 |
| Count Values to Serum (Average) | Serum | 100% | 100% | 100% | 100% |
| | Heparin Plasma | 165% | 100% | 99% | 100% |
| | EDTA Plasma | 780% | 112% | 109% | 112% |
| | Citrate Plasma | 185% | 100% | 98% | 101% |

As a result, when cardiac troponin I in the test samples were measured in the absence of dextran sulfate sodium, the measurement values were largely varied among serums, EDTA plasmas, citrate plasmas, and heparin plasmas (Table 1, and FIGs. 1 to 3). On the other hand, when cardiac troponin I was measured in the presence of dextran sulfate sodium by using an antibody-conjugated particle solution containing dextran sulfate sodium, measurement values of cardiac troponin I in the test samples were almost identical among serums, EDTA plasmas, citrate plasmas, and heparin plasmas (Table 1, and FIGs. 1 to 3).

From the above, it was revealed that a difference of measurement values of cardiac troponin I between a serum and plasmas is reduced by measuring cardiac troponin I in the presence of dextran sulfate sodium.

### [Example 3] Effects of Type of Antibody Epitope to Correlation between Serum and Plasma on Measurement of Cardiac Troponin I in the Presence of Dextran Sulfate Sodium

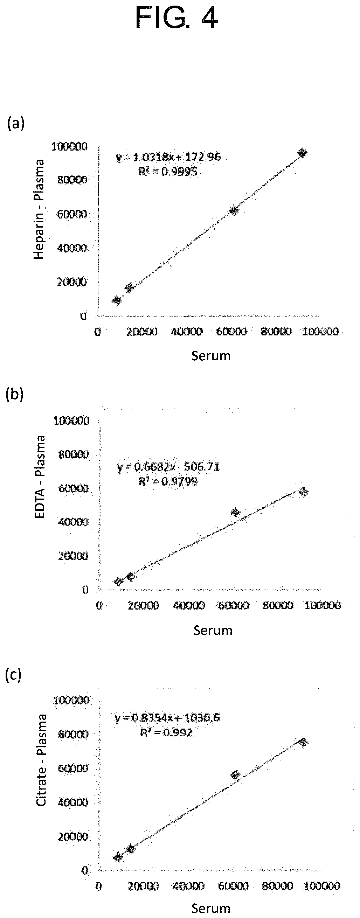

Dextran sulfate sodium (Dextran sulfate sodium 5000, manufactured by Wako Pure Chemical Industries, Ltd.) was added to an antibody-conjugated particle solution of Reagent B so that the concentration was 0 mg/mL, 0.5 mg/mL, 1.0 mg/mL, or 2.0 mg/mL (each of the concentrations in the first reaction solution was 0 mg/mL, 0.3 mg/mL, 0.6 mg/mL, 1.2 mg/mL), and then the test samples were measured. Other conditions were same to those in Example 2. The results are listed in Table 2 and illustrated in FIGs. 4 to 7. Each of the measurement values is a luminescence intensity at a wavelength of 477 nm (count value).

**Table 2. Measurement of Cardiac Troponin I Using Reagent B**

| | | Dextran Sulfate Sodium Content (mg/mL) | | | |
|---|---|---|---|---|---|
| Sample | Sample Type | 0 | 0.5 | 1.0 | 2.0 |
| F | Serum | 8808 | 7859 | 7523 | 7347 |
| | Heparin Plasma | 8957 | 7724 | 7367 | 7241 |
| | EDTA Plasma | 4705 | 7156 | 7220 | 6641 |
| | Citrate Plasma | 7665 | 7781 | 7630 | 7354 |
| G | Serum | 14775 | 13234 | 12721 | 12454 |
| | Heparin Plasma | 16230 | 13974 | 12974 | 12574 |
| | EDTA Plasma | 7914 | 12278 | 12154 | 12022 |
| | Citrate Plasma | 12448 | 13648 | 16205 | 12947 |
| H | Serum | 60856 | 56741 | 54938 | 54538 |
| | Heparin Plasma | 61753 | 57897 | 57765 | 62060 |
| | EDTA Plasma | 45574 | 58139 | 57935 | 55097 |
| | Citrate Plasma | 56120 | 59534 | 58829 | 55748 |
| I | Serum | 91814 | 82199 | 81794 | 80285 |
| | Heparin Plasma | 95602 | 85201 | 82625 | 76809 |
| | EDTA Plasma | 57548 | 80313 | 81216 | 76354 |
| | Citrate Plasma | 75140 | 76111 | 74281 | 73653 |
| Count Values to Serum (Average) | Serum | 100% | 100% | 100% | 100% |
| | Heparin Plasma | 104% | 102% | 102% | 102% |
| | EDTA Plasma | 61% | 96% | 99% | 96% |
| | Citrate Plasma | 86% | 100% | 107% | 100% |

As a result, even when using Reagent B containing a pair of antibodies (an immobilized antibody and a labeled antibody) recognizing a portion of cardiac troponin I antigen, which portion is different from that recognized by the pair of antibodies (an immobilized antibody and a labeled antibody) contained in Reagent A used in Example 2, measurement values of cardiac troponin I in the test samples were almost identical among serums, EDTA plasmas, citrate plasmas, and heparin plasmas in the presence of dextran sulfate sodium using an antibody-conjugated particle solution containing dextran sulfate sodium (Table 2, and FIGs. 4 to 7).

From the above, it was revealed that, regardless of the position of cardiac troponin I epitope recognized by the antibody, a difference of measurement values of cardiac troponin I between a serum and plasmas is reduced by measuring cardiac troponin I in the presence of dextran sulfate sodium.

### [Example 4] Effects of Various Polyanionic Macromolecule to Correlation between Serum and Plasma on Measurement of Cardiac Troponin I

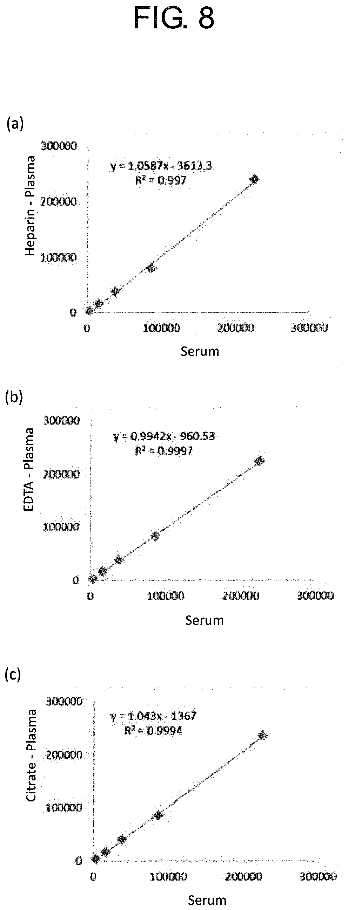

Dextran sulfate sodium (Dextran sulfate sodium 5000, manufactured by Wako Pure Chemical Industries, Ltd.), sodium polystyrene sulfonate (weight-average molecular weight: ∼70,000, manufactured by Sigma Aldrich Corporation), sodium polyacrylate (weight-average molecular weight: ∼5,100, manufactured by Sigma Aldrich Corporation), sodium N-lauroyl sarcosinate (molecular weight 271, manufactured by Nacalai Tesque, Inc.), or L-aspartic acid (molecular weight 133, manufactured by Wako Pure Chemical Industries, Ltd.) was added to an antibody-conjugated particle solution of Reagent B so that the concentration was 1.5 mg/mL (the concentration in the first reaction solution was 0.9 mg/mL), and then the test samples were measured. Other conditions were same to those in Examples 2 and 3. The results are listed in Table 3 and illustrated in FIGs. 8 to 11. Each of the measurement values is a luminescence intensity at a wavelength of 477 nm (count value).

**Table 3. Measurement of Cardiac Troponin I in the Presence of Polyanionic Macromolecule**

| | | Polyanionic Macromolecule | | | | | |
|---|---|---|---|---|---|---|---|
| Sample | Sample Type | None | a | b | c | d | e |
| J | Serum | 6326 | 3907 | 1608 | 6674 | 4664 | 5212 |
| | Heparin Plasma | 5615 | 3347 | 1464 | 5504 | 4352 | 5234 |
| | EDTA Plasma | 4158 | 3223 | 1461 | 5447 | 3464 | 3841 |
| | Citrate Plasma | 5431 | 3680 | 1502 | 6130 | 4082 | 3978 |
| K | Serum | 17754 | 17312 | 7992 | 21247 | 14458 | 13552 |
| | Heparin Plasma | 18599 | 17536 | 8367 | 20787 | 15914 | 18714 |
| | EDTA Plasma | 13750 | 17020 | 8004 | 19694 | 11636 | 9780 |
| | Citrate Plasma | 16146 | 17721 | 8604 | 22274 | 12638 | 8940 |
| L | Serum | 40805 | 39011 | 20730 | 52734 | 33996 | 34473 |
| | Heparin Plasma | 42770 | 38030 | 19354 | 47267 | 34609 | 42669 |
| | EDTA Plasma | 31453 | 38707 | 20273 | 45254 | 29169 | 26912 |
| | Citrate Plasma | 40896 | 40128 | 21199 | 46781 | 31740 | 24736 |
| M | Serum | 92676 | 87524 | 41330 | 101754 | 74472 | 62083 |
| | Heparin Plasma | 89762 | 79811 | 39574 | 93448 | 73140 | 87331 |
| | EDTA Plasma | 57851 | 83219 | 41458 | 96826 | 51929 | 44569 |
| | Citrate Plasma | 67722 | 85718 | 42532 | 105823 | 56370 | 38698 |
| N | Serum | 267504 | 226173 | 119764 | 283544 | 204993 | 175426 |
| | Heparin Plasma | 285814 | 239089 | 121290 | 291650 | 224399 | 273378 |
| | EDTA Plasma | 175701 | 224771 | 118838 | 243719 | 149783 | 122908 |
| | Citrate Plasma | 233473 | 235920 | 125361 | 278351 | 177374 | 113692 |
| Count Values to Serum (Average) | Serum | 100% | 100% | 100% | 100% | 100% | 100% |
| | Heparin Plasma | 100% | 96% | 97% | 93% | 103% | 132% |
| | EDTA Plasma | 70% | 95% | 98% | 88% | 77% | 73% |
| | Citrate Plasma | 87% | 100% | 102% | 97% | 86% | 68% |

| | | | | | | | |
|---|---|---|---|---|---|---|---|
| a: Dextran sulfate sodium b: Sodium polystyrene sulfonate c: Sodium polyacrylate d: Sodium N-lauroyl sarcosinate e: L-Aspartate | | | | | | | |

As a result, when cardiac troponin I was measured in the presence of dextran sulfate sodium, sodium polystyrene sulfonate, or sodium polyacrylate (a polyanionic macromolecule), measurement values of cardiac troponin I in the test samples were almost identical among serums, EDTA plasmas, citrate plasmas, and heparin plasmas (Table 3, and FIGs. 8 to 11). On the other hand, a difference of measurement values of cardiac troponin I between the serum and each of the plasmas was not reduced in the presence of sodium N-lauroyl sarcosinate and L-aspartate, which are low molecular weight anionic compounds (low molecular weight compounds containing anionic portions).

From the above, it was revealed that a difference of measurement values between the serum and each of the plasmas is reduced by measuring cardiac troponin I in the presence of a polyanionic macromolecule.

## Claims

1. A reagent for measuring cardiac troponin I, comprising an antibody against cardiac troponin I and a polyanionic macromolecule.

2. The reagent according to claim 1, wherein the polyanionic macromolecule contains a group selected from the group consisting of a sulfate group, a sulfonate group, and a carboxylate group.

3. The reagent according to claim 1 or 2, wherein the reagent comprises a solution containing the antibody and the macromolecule.

4. The reagent according to claim 3, wherein the macromolecule in the solution has a concentration of 0.06 mg/mL or higher but 85 mg/mL or lower.

5. The reagent according to any one of claims 1 to 4, wherein the antibody is an immobilized antibody.

6. The reagent according to any one of claims 1 to 5, further comprising an additional antibody against cardiac troponin I.

7. The reagent according to claim 6, wherein the additional antibody is a labeled antibody.

8. A method for measuring cardiac troponin I, comprising measuring an amount of cardiac troponin I in a blood sample by using an antibody against cardiac troponin I in the presence of a polyanionic macromolecule.

9. The method according to claim 8, wherein the blood sample is plasma.

10. The method according to claim 8 or 9, comprising (1) to (3) below:
(1) preparing a mixed solution of the antibody against cardiac troponin I, the polyanionic macromolecule, and the blood sample;
(2) incubating the mixed solution; and
(3) measuring an amount of cardiac troponin I in the mixed solution.

11. The method according to claim 10, wherein the macromolecule in the mixed solution has a concentration of 0.05 mg/mL or higher but 5.0 mg/mL or lower.
